(19) Europäisches Patentamt
European Patent Office
Office européen des brevets

(11) Numéro de publication: **0 312 432 B1**

(12) **FASCICULE DE BREVET EUROPEEN**

(45) Date de publication de fascicule du brevet: **12.01.94**

(51) Int. Cl.5: **C07D 405/04**, C07F 9/547, A61K 31/44, A61K 31/675

(21) Numéro de dépôt: **88402548.7**

(22) Date de dépôt: **10.10.88**

Demande divisionnaire 93106947.0 déposée le 10/10/88.

Le dossier contient des informations techniques présentées postérieurement au dépôt de la demande et ne figurant pas dans le présent fascicule.

# (54) Dérivés du diméthyl-2,2 chromène, procédé d'obtention et compositions/pharmaceutiques en contenant.

(30) Priorité: **12.10.87 FR 8714067**

(43) Date de publication de la demande: **19.04.89 Bulletin 89/16**

(45) Mention de la délivrance du brevet: **12.01.94 Bulletin 94/02**

(84) Etats contractants désignés: **AT BE CH DE ES FR GB GR IT LI LU NL SE**

(56) Documents cités: **EP-A- 0 076 075** **EP-A- 0 273 262**

(73) Titulaire: **ELF SANOFI**
**32-34, rue Marbeuf**
**F-75008 Paris(FR)**

(72) Inventeur: **Garcia, Georges**
**27, Rue des Aires**
**F-34980 Saint-Gely-du-Fesc(FR)**
Inventeur: **Di Malta, Alain**
**170, Rue de Vigne Belle**
**/**
**St Clément la Rivière**
**F-34980 Saint-Gely-du-Fesc(FR)**
Inventeur: **Gautier, Patrick**
**Lieu dit Manavieille**
**F-34660 Cournonterral(FR)**

(74) Mandataire: **Gillard, Marie-Louise et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet des dérivés du chromène à activité antihypertensive et antiarythmique. Elle concerne également un procédé pour leur préparation et les compositions pharmaceutiques les contenant.

Le brevet belge 829 611 mentionne toute une série de dérivés du chromannol-3 à activité antihypertensive ; ces dérivés sont caractérisés par la présence en position 4 d'un groupe $NR_1R_2$ dans lequel $R_1$ est l'hydrogène ou un groupe hydrocarboné éventuellement substitué, $R_2$ est l'hydrogène ou un alkyle, $NR_1R_2$ pouvant être un groupe hétérocyclique comportant de 3 à 8 atomes, non substitué ou substitué par un ou deux groupes méthyles et par la présence éventuelle d'un grand nombre de substituants possibles en position 6 ou en position 7.

La demande de brevet européen publiée sous le numéro 76 075 décrit des dérivés du chromannol-3 à activité antihypertensive caractérisés par la présence en position 4 d'un groupe oxo-2 pyrrolidinyl-1 ou d'un groupe oxo-2 pipéridino et par la présence éventuelle de nombreux substituants possibles, dont le groupe cyano, en position 6 ou en position 7.

La demande de brevet européen 93535 décrit des dérivés du diméthyl-2,2 chromène caractérisés par la présence en position 4 d'un groupe oxo-2 pipéridino ou oxo-2 pyrrolidinyl-1 et par la présence éventuelle de nombreux substituants possibles en position 6 ou en position 7.

Par ailleurs, un article paru dans J. Med. Chem., 1986 29, 2194-2201, compare les activités antihypertensives des dérivés du chromannol et des dérivés du chromène correspondants décrits dans les 2 demandes de brevet européens citées ci-dessus.

Les résultats obtenus pour la diminution de la pression sanguine chez le rat spontanément hypertendu, montrent que les dérivés du chromannol présentent des activités semblables à celles des dérivés du chromène correspondant.

La demanderesse a trouvé que les dérivés du diméthyl-2,2 chromène caractérisés par la présence, en position 4, d'un groupe oxo-2 dihydro-1,2 pyridyl-1 possèdent une excellente activité antihypertensive et antiarythmique et une toxicité très faible.

De façon tout-à-fait surprenante, lesdits dérivés présentent une activité antihypertensive supérieure à celle des dérivés du chromannol-3 correspondant.

La demande de brevet EP-A-0 273 262, publiée le 6 juillet 1988 - déposée en désignant les états contractants ci-après : AT-BE-CH-DE-ES-FR-GB-IT-LI-NL-SE - décrit elle aussi des dérivés du chromanne, actifs sur le système cardiovasculaire.

Etant donné l'existence de ce document, la présente invention concerne des dérivés du diméthyl-2,2 chromène, de formule

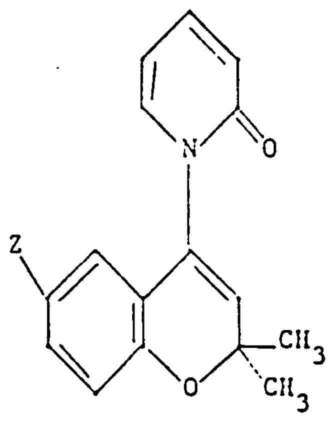

(I)

dans laquelle :

- pour les états contractants LU et GR, Z représente un groupement cyano, phosphono ou dialcoxyphosphoryle, le groupe alcoxy contenant 1 à 3 atomes de carbone;
- pour les états contractants AT, BE, CH, DE, ES, FR, GB, IT, LI, NL, SE, Z représente un groupement phosphono ou dialcoxyphosphoryle, le groupe alcoxy contenant 1 à 3 atomes de carbone;

et les sels pharmaceutiquement acceptables du groupe phosphono.

Les sels pharmaceutiquement acceptables sont de préférence ceux des métaux alcalins et alcalino-terreux tels que les sels de sodium et de potassium ou ceux des bases organiques comme la triéthanolamine, le trométamol, l'éthanolamine, la N-méthylpipéridine ou la ter-butylamine.

La présente invention a également pour objet un procédé pour la préparation des composés (I).

Ledit procédé est caractérisé en ce que l'on déshydrate le chromannol-3 de formule :

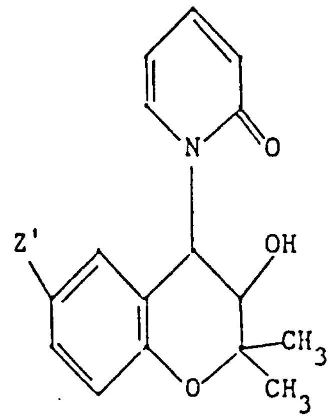

(II)

dans laquelle Z' représente un groupe cyano, ou dialcoxyphosphoryle et en ce que l'on transforme éventuellement le groupe dialcoxyphosphoryle en le groupe phosphono, puis en ce que l'on transforme éventuellement l'acide phosphonique ainsi obtenu en un de ses sels pharmaceutiquement acceptables. Le produit final de la reaction (I) est isolé selon les méthodes classiques.

Lorsque Z' représente un groupe dialcoxyphosphoryle, celui-ci peut être transformé en le groupe phosphono correspondant par transestérification avec un halogénure de triméthylsilyle, de préférence le bromure, et hydrolyse du di (triméthylsilylester) par simple action de l'eau. On obtient ainsi un composé de formule I, dans laquelle Z représente un groupe phosphono, et ledit composé peut être transformé dans un de ses sels pharmaceutiquement acceptables, par exemple ceux de métaux alcalins ou alcalino-terreux tels que les sels de sodium ou de potassium ou ceux des bases organiques comme la triéthanolamine, le trométamol, l'éthanolamine, la tert-butylamine ou la N-méthylpipéridine.

La déshydratation du chromannol-3 est effectuée par un hydrure alcalin, tel que l'hydrure de sodium, dans un solvant inerte, tel que le tétrahydrofuranne, à une température comprise entre 50 °C et 100 °C.

Pour préparer le chromannol-3 (II), on traite un époxyde de chromanne de formule :

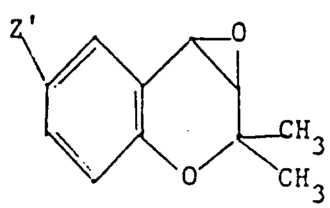

(III)

dans laquelle Z' est tel que défini ci-dessus avec l'hyroxy-2 pyridine.

La réaction d'ouverture de l'époxyde III est conduite à une température comprise entre 10 et 100 °C dans un solvant organique inerte comme le dioxanne, le tétrahydrofuranne, le méthyl-tertbutyléther, le diméthylsulfoxide ou le diméthylformamide en présence d'un agent de condensation basique tel que l'hydrure de sodium, un hydroxyde d' ammonium quaternaire comme l'hydroxyde de benzyl triméthyl ammonium. Dans ces conditions opératoires, l'ouverture de l'époxyde III conduit à un dérivé chromannol-3 de configuration trans.

Les époxydes de départ de formule III sont connus ou préparés selon des méthodes connues. Ainsi l'époxyde III dans lequel Z' représente le groupe cyano est décrit dans le brevet belge 852 955 :

Les époxydes de départ de formule III, dans laquelle Z' représente un groupe dialcoxyphosphoryle ne sont pas décrits en littérature. Ils peuvent être préparés à partir du bromo-6 diméthyl-2,2 chromène (J.Chem.Soc. 1960, 3094-3098) de formule :

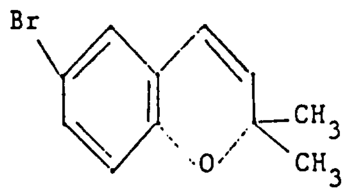

(IV)

par action d'un trialkylphosphite en présence de chlorure de nickel à 180 ° C, et réaction du composé ainsi obtenu de formule :

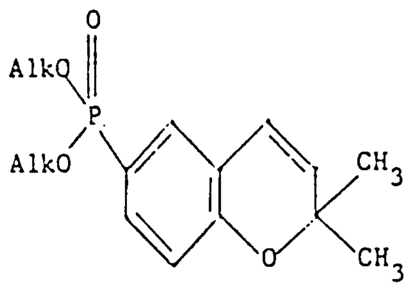

(V)

dans laquelle Alk représente un alkyle contenant de 1 à 3 atomes de carbone, avec le N-bromosuccinimide dans du diméthylsulfoxyde aqueux.

La bromhydrine ainsi obtenue, de formule :

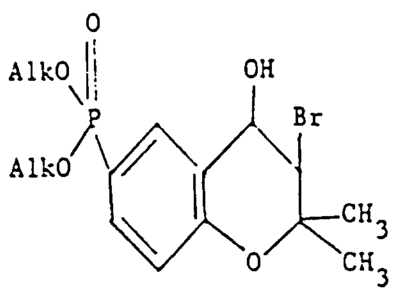

(VI)

dans laquelle Alk est tel que défini ci-dessus, est ensuite traitée avec un agent alcalin dans un mélange eau/solvant organique, par exemple eau/dioxanne de préférence à la température ambiante pendant une période de 8 à 20 heures et l'époxyde ainsi obtenu de formule II, dans laquelle Z' est un groupe dialcoxyphosphoryle, est isolé selon les méthodes classiques, par exemple par concentration du mélange réactionnel et récupération du résidu avec un solvant qui élimine les impuretés, tel que le chlorure de méthylène, lavage à l'eau et concentration.

Les composés de formule I augmentent la polarisation des fibres musculaires lisses et ont un effet vasodilateur sur la veine porte; leur effet antihypertenseur a été observé chez l'animal.

Par ailleurs, on a observé que les composés selon l'invention accélèrent la repolarisation des cellules myocardiques ; parallèlement, leur effet antiarythmique a été observé sur un modèle animal.

Aucun signe de toxicité n'est observé avec ces composés aux doses pharmacologiquement actives.

Ainsi les composés selon l'invention peuvent être utilisés dans le traitement de l'hypertension, des troubles pathologiques associés aux contractions des fibres musculaires lisses des appareils gastrointestinal, respiratoire, utérin et urinaire, par exemple : ulcère, asthme, contraction utérine prématurée, incontinence, et dans le traitement d'autres troubles pathologiques cardiovasculaires tels que : angor, insuffisance cardiaque, maladies vasculaires cérébrales et périphériques. De plus, les composés selon l'invention peuvent être utilisés dans le traitement de l'arythmie cardiaque. Enfin, les composés de la présente invention peuvent être utilisés pour le traitement topique de l'alopécie.

La présente invention a également pour objet des compositions pharmaceutiques contenant une dose efficace d'un composé selon l'invention avec des excipients convenables. Lesdits excipients sont choisis selon la forme pharmaceutique et le mode d'administration souhaités.

Un autre objet de la présente invention consiste en le procédé de préparation de telles compositions pharmaceutiques. Une dose efficace d'un dérivé du diméthyl-2,2 chromène selon l'invention est mélangé à des excipients convenables.

Dans les compositions pharmaceutiques de la présente invention pour l'administration orale, sublinguale, sous-cutanée, intramusculaire, intraveineuse, topique, transdermique ou rectale, les principes actifs de formule I ci-dessus ou leurs sels éventuels peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale. Pour l'application topique, on peut utiliser les compositions selon l'invention dans des crèmes, pommades ou lotions.

Afin d'obtenir l'effet prophylactique ou thérapeutique désiré, la dose de principe actif peut varier entre 0.01 et 5 mg par kg de poids du corps et par jour.

Chaque dose unitaire peut contenir de 0,5 à 200 mg, de préférence de 1 à 50 mg, d'ingrédients actifs en combinaison avec un support pharmaceutique. Cette dose unitaire peut être administrée 1 à 5 fois par jour de façon à administrer un dosage journalier de 0,5 à 1000 mg, de préférence de 5 à 250 mg.

Lorsqu'on prépare une composition solide sous forme de comprimés, on mélange le principe actif principal avec un véhicule pharmaceutique tel que la gélatine, l'amidon, le lactose, le stéarate de magnésium, le talc la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir ou pour l'administration sous forme de gouttes peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule I ci-dessus ou d'un de leurs sels pharmaceutiquement acceptables, d'autres principes actifs tels que, par exemple, des tranquillisants ou d'autres médicaments qui peuvent être utiles dans le traitement des troubles ou maladies indiqués ci-dessus.

Les exemples suivants illustrent l'invention. Dans les exemples, ainsi que dans la partie descriptive et dans les revendications, les produits sont désignés conme dérivés du chromanne. Il est entendu que les produits de la présente invention sont des dérivés du diméthyl-2,2 2H-chromène et que les termes "chromène" et "chromanne" désignent respectivement le "2H-chromène" et le "dihydro-3,4 2H-chromène".

## PREPARATION DU DIETHYLPHOSPHONO-6 DIMETHYL-2,2 EPOXY-3,4 CHROMANNE

A) Diéthylphosphono-6 diméthyl-2,2 2H-chromène

16 g de bromo-6 diméthyl-2,2 2H-chromène sont dissous dans 100 ml de triéthylphosphite. On ajoute 2 g de chlorure de nickel et on chauffe à reflux à 180 ° C pendant 24 heures dans un autoclave. Après avoir concentré le triéthylphosphite restant, le produit attendu distille à 130-140 ° C sous 0,1 mm de Hg. On recueille 11,5 g.

B) trans-Bromo-3 diéthylphosphono-6 diméthyl-2,2 chromannol-4

11 g du produit précédent sont dissous dans 62 ml de diméthylsulfoxide contenant 1,35 ml d'eau. En Maintenant la solution à une température inférieure à 20 ° C, on ajoute par petites fractions 12,2 g de N-bromosuccinimide. On laisse sous agitation à température ambiante pendant 30 minutes puis on ajoute 100 ml d'eau et on extrait à l'acétate d'éthyle.Après séchage sur sulfate de sodium, on concentre la phase organique, reprend le résidu par 100 ml d'acétone et 50 ml d'eau, puis on chauffe au reflux pendant 5 heures. On concentre l'acétone, extrait à l'éther, sèche sur sulfate de sodium et concentre. Le produit attendu cristallise dans l'éther isopropylique. Après filtration et séchage des cristaux on recueille 3,2 g de produit. Point de fusion : 124 ° C.

C) Diéthylphosphono-6 diméthyl-2,2 époxy-3,4 chromanne

On mélange 23 g du composé obtenu à l'étape précédente et 12 g de soude dans 900 ml de dioxanne et 100 ml d'eau. Après 24 heures à température ambiante, on concentre le dioxanne, on reprend le résidu par de l'eau et extrait à l'éther éthylique puis sèche sur sulfate de sodium. Après concentration, on obtient 16,2 g du produit attendu.

EXEMPLE 1

Cyano-6 (dihydro-1,2 oxo-2 pyridyl-1)-4 diméthyl-2,2 chromène : SR 44866.

A) trans-Cyano-6 (dihydro-1,2 oxo-2 pyridyl-1)-4 diméthyl-2,2 chromannol-3

On chauffe à reflux pendant 40 heures 1 g de cyano-6 diméthyl-2,2 époxy-3,4 chromanne avec 1 g d'hydroxy-2 pyridine dans 10 ml de dioxanne en présence de 0,20 ml d'une solution méthanolique contenant 35 % d'hydroxyde de benzyl triméthylammonium. On reprend par 30 ml d'eau, le précipité obtenu est essoré, lavé par l'éther isopropylique puis cristallisé dans 20 ml d'alcool éthylique absolu. On obtient 0,9 g du produit attendu. Point de fusion : 243 ° C avec décomposition (tube capillaire).

B) SR 44866

On porte au reflux pendant 2 heures un mélange contenant 1,7 g du produit obtenu à l'étape A et 150 mg d'hydrure de sodium dans 50 ml de tétrahydrofuranne. On évapore le tétrahydrofuranne sous vide, on reprend le résidu par de l'eau glacée, puis on extrait à l'acétate d'éthyle. La phase organique est séchée sur sulfate de sodium puis évaporée sous vide. Le résidu est repris dans un mélange éther isopropylique-éther éthylique (50/50,v/v). Après avoir filtré, lavé à l'éther isopropylique et séché à 100 ° C sous vide, on recueille 800 mg du produit attendu. Point de fusion : 151 ° C.

EXEMPLE 2

Diéthylphosphono-6 (dihydro-1,2 oxo-2 pyridyl-1)-4 diméthyl-2,2 chromène : SR 45014.

A) trans-Diéthyphosphono-6 (dihydro-1,2 oxo-2 pyridyl-1)-4 diméthyl-2,2 chromannol-3

On chauffe à reflux pendant 48 heures un mélange contenant 5,6 g de diéthylphosphono-6 diméthyl-2,2 époxy-3,4 chrommane, 2,5 g d'hydroxy-2 pyridine et 4 gouttes d'hydroxyde de benzyl triméthylammoniun. Après avoir concentré le dioxanne, on reprend le résidu dans du dichlorométhane, lave deux foix à l'eau puis sèche sur sulfate de sodium et concentre à sec. On ajoute de l'éther éthylique, le produit cristallise. Après recristallisation dans l'acétate d'éthyle, on obtient 1,5 g du produit attendu. Point de fusion : 130 ° C (tube capillaire).

B) SR 45014

2 g du chromannol-3 obtenu à l'étape précédente sont dissous dans 100 ml de tétrahydrofuranne. On ajoute par petites fractions 120 mg d'hydrure de sodium puis on chauffe à reflux pendant 8 heures. Après avoir concentré à sec le milieu réactionnel, on extrait 3 fois par du chlorure de méthylène. La phase organique est séchée et concentrée, l'huile obtenue est purifiée par chromatographie sur une colonne de

silice en éluant par un mélange chlorure de méthylène-méthanol (98/2,v/v). On obtient 200 mg d'un produit cireux caractérisé par son spectre de RMN et son spectre IR.

Le spectre IR est enregistré pour le produit à 2 % dans le chlorure de méthylène : 980 cm -1. (C-O-C), 1030 cm -1. et 1055 cm -1. (P-O-C), 1240 cm -1. (P = O), 1490 cm -1., 1540 cm -1. et 1600 cm -1. (C = C), 1670 cm -1. (C = O), 2960 cm -1. 2970 cm -1., 2990 cm -1. (C-H).

Le spectre de RMN est enregistré à 250 MHz, en solution dans le DMSO.

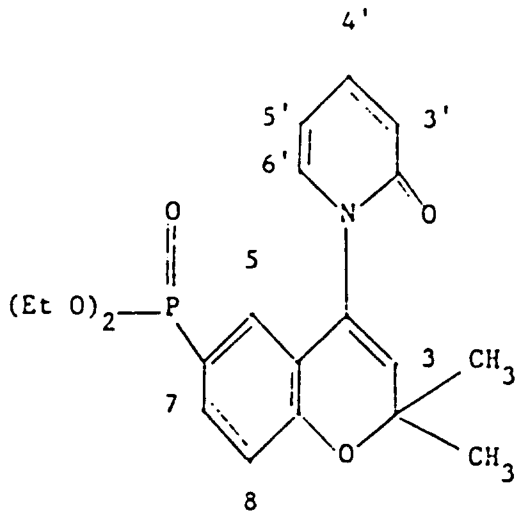

SPECTRE DE RMN

| Delta | Aspect | Protons | Attribution |
|---|---|---|---|
| 1,1ppm | T de D | 6H | 2 $CH_3$ (Et) |
| | J $CH_3$,$CH_2$ = 8Hz | | |
| | J $CH_3$,P = 2Hz | | |
| 1,45 et 1,5 ppm | 2 S | 6H | $(CH_3)2C$ |
| 3,83ppm | M | 4H | 2 $CH_2$ (Et) |
| 6,05ppm | S | 1H | H3 |
| 6,3 ppm | T de D | 1H | H5' |
| | Jo5',6'=7Hz | | |
| | Jm5',3'=1,25Hz | | |
| 6,45ppm | D de D | 1H | H3' |
| | Jo3'4'=10Hz | | |
| | Jm3'5'=1,25Hz | | |
| 6,77ppm | D de D | 1H | H5 |
| | J5,P=13Hz | | |
| | J5,7=1,5Hz | | |
| 6,95ppm | D de D | 1H | H8 |
| | Jo8,7=8Hz | | |
| | J 8,P=3Hz | | |
| 7,45ppm | D de D de D | 1H | H7 |

| | | | | |
|---|---|---|---|---|
| | J 7,P=13Hz | | | |
| | Jo7,8=8Hz | | | |
| | Jm7,5=1,5Hz | | | |
| | | | | |
| 7,53ppm | T de D | 1H | H4' | |
| | Jo4',3'=7Hz | | | |
| | Jo4',5'=7Hz | | | |
| | Jm4',6'=1,8Hz | | | |
| | | | | |
| 7,56ppm | D | 1H | H6' | |
| | Jo6',5'=7Hz | | | |
| | | | | |

S signifie Singulet
D signifie Doublet
T signifie Triplet
M signifie Multiplet

J représente la constante de couplage

Des compositions pharmaceutiques contenant le produit selon l'invention ont été préparées.

EXEMPLE 3

Comprimé enrobé.

Des comprimés peuvent être préparés par granulation humide. On utilise comme solvant auxiliaire de fabrication l'alcool éthylique et l'eau purifiée. Après l'évaporation de ces solvants, le stéarate de magnésium intervient en phase externe comme lubrifiant. On procède ensuite à l'enrobage des comprimés.

| Formulation | |
|---|---|
| SR 44 866 | 1 mg |
| Alcool éthylique à 95 % | 0,02 ml |
| Cellulose microcristalline | 48 mg |
| Lactose | 69,8 mg |
| Stéarate de magnésium | 1,2 mg |
| Eau purifiée q.s.p. | 120 mg |

| Formule d'enrobage : | |
|---|---|
| Méthyl hydroxy propylcellulose 6 cps | 0,14 mg |
| Dioxyde de titane | 0,04 mg |
| Polyoxyéthylèneglycol 6000 | 0,02 mg |
| Eau purifiée | 1,8 mg |
| Talc pour un comprimé pelliculé q.s.p. enrobé à | 122 mg |

EXEMPLE 4

| Forme injectable. | |
|---|---|
| SR 44 866 | 1 mg |
| Polyoxyéthylène glycol 400 | 0,5 ml |
| Eau purifiée pour préparation injectable | qs pour 1 ml |

EXEMPLE 5

| Forme injectable. | |
|---|---|
| SR 44 866 | 1 mg |
| Polysorbate 80$^{R}$ | 0,1 ml |
| Propylène glycol | 0,1 g |
| Eau purifiée pour préparation injectable | qs pour 1 ml. |

Les produits selon l'invention ont été étudiés dans les tests de pharmacologie in vitro et in vivo A), B) et C) ci-dessous.

Comme composé de référence, on a utilisé les composés suivants :

- le trans-cyano-6 (oxo-2 pyrrolidinyl-1)-4 diméthyl-2,2 chromannol-3, décrit dans EP 76075:

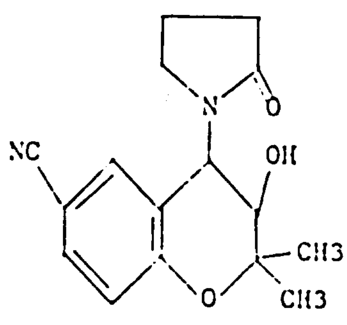

ci-après désigné "Produit A",

- le cyano-6 (oxo-2 pyrrolidinyl-1)-4 diméthyl-2,2 chromène, décrit dans la demande de brevet européen EP 95535, ci-après dénommé 'produit B".

On a également comparé l'activité des produits selon l'invention à celle du chromamnol-3 correspondant :

- le trans-cyano-6 (dihydro-1,2 oxo-2 pyridyl-1)-4 diméthyl-2,2 chromannol-3, préparé à l'exemple 1 (produit C).

A) Veine porte isolée de rat.

Les rats mâles Sprague Dawley (250 - 300 g) sont assommés puis saignés après section de la gorge. La veine porte, ligaturée in situ en deux endroits distants de 15 mm, est isolée et coupée longitudinalement

puis montée verticalement dans la cuve d'expérimentation contenant une solution physiologique à 37 ° C de composition suivante (mM) : NaCl : 137; KCl : 5,4; $MgCl_2$ : 1,05; $CaCl_2$ : 1,8; $NaH_2PO_4$ : 1,2; $NaHCO_3$ : 15,5; Glucose 11,5 dans laquelle on fait barbotter un mélange gazeux contenant 95 % d'oxygène et 5 % de gaz carbonique.

La veine est soumise à une tension de 500 mg. Après une période de stabilisation (environ 1 h 30) les activités contractiles spontanées sont enregistrées à l'aide d'un capteur isométrique. Chaque mesure est réalisée Successivement sur 4 préparations.

Le produit est étudié à concentrations croissantes et successives (15 min par concentration) jusqu'à inhibition totale des contractions spontanées. Les résultats sont exprimés sous forme de concentration molaire provoquant une inhibition de 50 pour cent (CI50) des activités contractiles spontanées.

Les résultats obtenus sont rassemblés dans le tableau 1 ci-dessous.

Dans la colonne des résultats, on a reporté la CI50 des activités contractiles spontanées de la veine porte isolée de rat.

TABLEAU 1

| Inhibition des activités contractiles spontanées. | | | | |
|---|---|---|---|---|
| Produit | Exemple | CI50 M | Comparaison | |
| | | | Produit | CI50 M |
| SR 44866<br>A | 1 | $7{,}1.\ 10^{-9}$<br>$6{,}8.\ 10^{-8}$ | C<br>B | $9{,}0.\ 10^{-8}$<br>$7{,}0.\ 10^{-8}$ |

Tous les composés étudiés présentent une importante activité inhibitrice des contractions spontanées de la veine, supérieure à celle des produits de référence.

On constate que chaque dérivé du chromène selon l'invention a une activité au moins 10 fois supérieure à celle du dérivé du chromannol-3 correspondant, tandis que les produits de comparaison A et B présentent des activités similaires.

B) Muscle papillaire de cobaye.

Les cobayes mâles Albinos Charles River (300 - 400 g) sont assommés puis saignés après section de la gorge. Le coeur est isolé et ouvert, le muscle papillaire droit est excisé puis maintenu en survie dans la cuve d'expérimentation contenant une solution physiologique à 36 ° C (composition décrite ci-dessus).

La préparation est, stimulée à l'aide d'une électrode bipolaire reliée à un stimulateur (fréquence = 60 battements par minute). Le potentiel d'action ventriculaire est recueilli selon la méthode classique de la microélectrode. Les paramètres caractéristiques ont été mesurés sur les potentiels d'action (PA) avant et après l'introduction, du produit à tester à 3 concentrations successives et croissantes (30 minutes de perfusion par concentration). La concentration produisant une réduction de 50 pour cent de la durée du PA est indiquée (CI50).

Les résultats sont reportés dans le tableau 2 ci-dessous :

TABLEAU 2

| Réduction de la Durée du potentiel d'action. | | |
|---|---|---|
| Produit | Exemple | CI50 |
| SR 44866<br>Produit A<br>Produit C | 1<br>-<br>- | $1{,}5.\ 10^{-6}$<br>$1{,}6.\ 10^{-5}$<br>$2{,}0.\ 10^{-5}$ |

Le tableau montre que la durée du potentiel d'action est nettement diminuée. Notamment, la concentration de SR 44866 produisant une réduction de 50 pour cent de ce paramètre est 10 fois inférieure à celle du produit A, démontrant une activité électrophysiologique supérieure sur la perméabilité membranaire responsable de cette phase de repolarisation.

Le profil électrophysiologique du composé étudié montre qu'il n'a pas d'effet significatif sur le potentiel de repos et la vitesse maximale de dépolarisaton, ceci signifie que le composé étudié n'a pas d'activité anesthésique locale.

C) Activité antihypertensive sur le rat spontanément hypertendu (SHR) vigile.

L'expérimentation est réalisée sur des rats SHR mâles (de souche Wistar) de 11 à 12 semaines, sous anesthésie au pentobarbital un cathéter est implanté la veille de l'expérimentation dans une artère carotide. Lors de l'expérimentation les pressions artérielles diastolique (PAD) et systolique (PAS) des animaux vigiles sont enregistrées en continu 1 heure avant et jusqu'à 2 heures après l'administration du produit. La fréquence cardiaque (FC) est déterminée à partir de la pression artérielle pulsatile et enregistrée en continu pendant le même temps.

Les produits ont été administrés par voie orale sous un volume de 2 ml pour 100 g de poids d'animal après mise en suspension dans une solution aqueuse de gomme arabique à 5 %.

Les résultats sont reportés dans le tableau 3 ci-dessous.

TABLEU 3

| Diminution de la pression artérielle. | | | | |
|---|---|---|---|---|
| Produit | Exemple | Dose mg/kg p.o. | Diminution maximale de la pression artérielle moyenne mm Mercure (± e.s.) | Durée de l'effet (minutes) |
| SR 44 866 | 1 | 0,02<br>0,03 | 34 ± 6<br>41 ± 6 | 90<br>> 120 |
| Produit A | - | 0,10<br>0,20 | 30 ± 10<br>56 ± 11 | 60<br>90 |

Les produits selon l'invention sont de puissants antihypertenseurs ayant une activité du même ordre ou supérieure à celle du Produit A.

De plus, on a constaté que les composés représentatifs de la présente invention possèdent une durée d'action plus longue que celle du Produit A.

Les produits de l'invention ont été également étudiés comme antiarythmiques dans le test D) ci-dessous.

D) Activité antiarythmique sur chien vigile.

La méthode utilisée est celle décrite par Dupuis et al., (Br. J. Pharmacol. 1976, 58, p. 409), dans laquelle un infarctus aigu est provoqué par l'insertion à thorax fermé d'une spire de cuivre dans la circulation coronaire. L'ECG est recueilli par télémétrie et les extrasystoles analysées et dénombrées automatiquement tandis que l'animal fait l'objet d'une surveillance par circuit de télévision interne. Les produits ont été administrés par voie orale chez des animaux présentant au moins 50 pour cent d'extrasystoles.

Un composé représentatif de la présente invention, le SR 44 866 administré per os à 0,03 mg/kg et 0,1 mg/kg montre une importante activité antiarythmique en réduisant le nombre d'extrasystoles ou en restaurant un rythme sinusal pendant une durée variant de 45 minutes à 2 heures selon les animaux.

Les données biologiques ci-dessus mettent en évidence que les composés selon l'invention sont des antihypertenseurs puissants et des antiarythmiques potentiels.

La toxicité aigue d'un produit représentatif de l'invention, le SR 44 866, a été mesurée chez la souris per os à différentes doses, pour un lot de 10 animaux, en comparaison avec le Produit A.

Les doses létales (DL) ont été calculées pour les 2 produits et sont reportées dans le tableau 4 ci-dessous.

TABLEAU 4

| | $DL_0$ mg/kg | $DL_{50}$ mg/kg |
|---|---|---|
| SR 44 866 | 500 | 1 000 |
| Produit A | 500 | entre 500 et 1 000 |

Ainsi, les 2 produits ont une toxicité comparable, alors que le SR 44866 présente une activité environ 10 fois supérieure dans la plupart des tests. En conclusion, les produits selon l'envention ont un coefficient thérapeutique supérieur à celui du produit de référence.

**Revendications**

**Revendications pour les Etats contractants suivants : AT, BE, CH, DE, FR, GB, IT, LI, NL, SE**

**1.** Dérivés du diméthyl-2,2 chromène de formule :

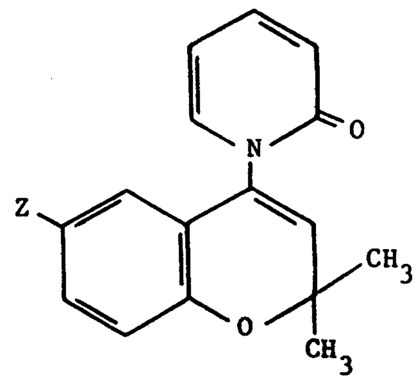

(I)

dans laquelle Z représente un groupement phosphono ou dialcoxyphosphoryle, le groupe alcoxy contenant 1 à 3 atomes de carbone ; et les sels pharmaceutiquement acceptables du groupe phosphono.

**2.** Le diéthylphosphono-6 (dihydro-1,2 oxo-2 pyridyl-1)-4 diméthyl-2,2 chromène.

**3.** Procédé de préparation d'un dérivé du chromène selon la revendication 1, caractérisé en ce que l'on déshydrate un chromannol-3 de formule :

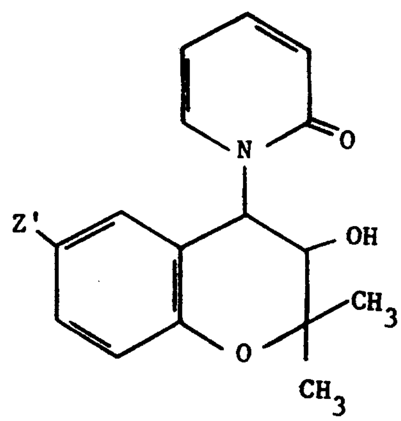

(II)

dans laquelle

- Z' représente un groupe dialcoxyphosphoryle, par un hydrure alcalin et en ce qu'on transforme éventuellement le groupe dialcoxyphosphoryle en le groupe phosphono puis en ce qu'on

transforme éventuellement l'acide phosphonique ainsi obtenu en un de ses sels pharmaceutiquement acceptables.

**4.** Composition pharmaceutique caractérisée en ce qu'elle contient, comme principe actif, un dérivés de diméthyl-2,2 chromène selon l'une des revendications 1 ou 2.

**5.** Composition pharmaceutique selon la revendication 4 contenant de 0,5 à 200 mg de principe actif par unité de dosage en mélange avec un excipient pharmaceutique.

**Revendications pour l'Etat contractant suivant : LU**

**1.** Dérivés du diméthyl-2,2 chromène de formule :

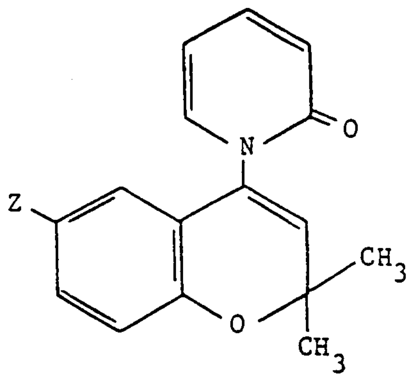

(I)

dans laquelle Z représente un groupement cyano, phosphono ou dialcoxyphosphoryle, le groupe alcoxy contenant 1 à 3 atomes de carbone; et les sels pharmaceutiquement acceptables du groupe phosphono.

**2.** Le cyano-6 (dihydro-1,2 oxo-2 pyridyl-1)-4 diméthyl-2,2 chromène.

**3.** Procédé de préparation d'un dérivé du chromène selon la revendication 1, caractérisé en ce que l'on déshydrate un chromannol-3 de formule :

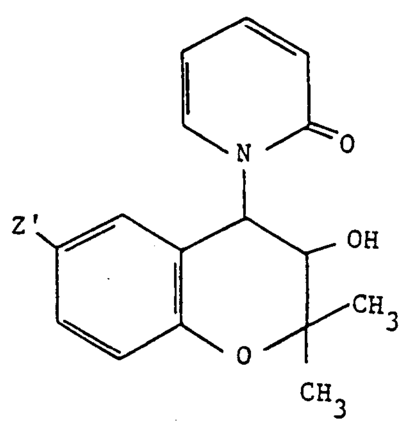

(II)

dans laquelle

- Z' représente un groupe cyano ou dialcoxyphosphoryle, par un hydrure alcalin et en ce qu'on transforme éventuellement le groupe dialcoxyphosphoryle en le groupe phosphono puis en ce qu'on transforme éventuellement l'acide phosponique ainsi obtenu en un de ses sels pharmaceutiquement acceptables.

**4.** Composition pharmaceutique caractérisée en ce qu'elle contient, comme principe actif, un dérivé de diméthyl-2,2,chromène selon l'une des revendications 1 ou 2.

**5.** Composition pharmaceutique selon la revendication 4 contenant de 0,5 à 200 mg de principe actif par unité de dosage en mélange avec un exipient pharmaceutique.

**Revendications pour l'Etat contractant suivant : ES**

**1.** Procédé de préparation de dérivés du diméthyl-2,2 chromène de formule :

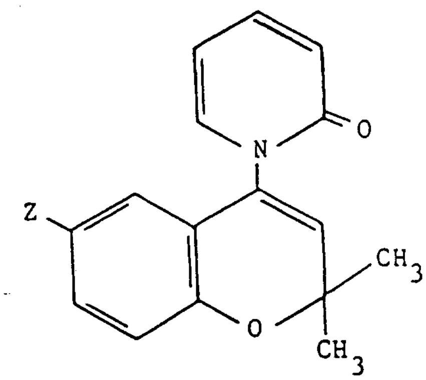

(I)

dans laquelle Z représente un groupement phosphono ou dialcoxyphosphoryle, le groupe alcoxy contenant 1 à 3 atomes de carbone;
et les sels pharmaceutiquement acceptables du groupe phosphono, caractérisé en ce que l'on déshydrate un chromannol-3 de formule :

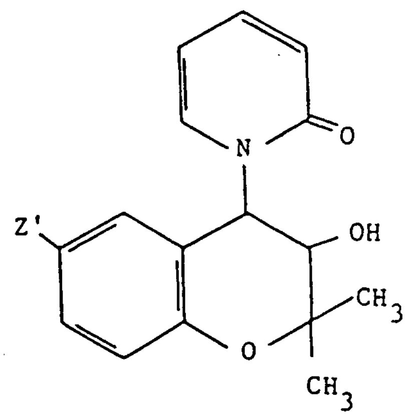

(II)

dans laquelle

- Z' représente un groupe dialcoxyphosphoryle, par un hydrure alcalin et en ce qu'on transforme éventuellement le groupe dialcoxyphosphoryle en le groupe phosphono puis en ce qu'on transforme éventuellement l'acide phosponique ainsi obtenu en un de ses sels pharmaceutiquement acceptable.

**2.** Procédé selon la revendication 1, pour la préparation du diéthylphosphono-6 (dihydro-1,2 oxo-2 pyridyl-1)-4 diméthyl-2,2 chromène, caractérisé en ce qu'il consiste à déshydrater avec de l'hydrure de sodium le chromannol-3 de formule :

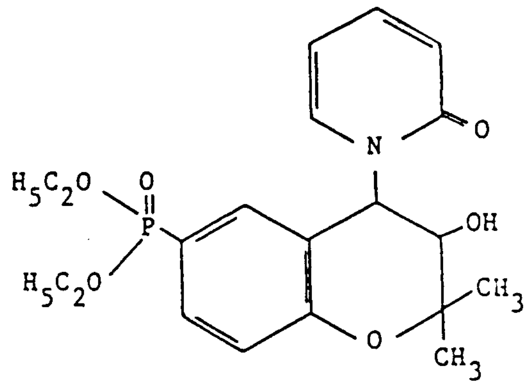

3. Procédé pour la préparation de compositions pharmaceutiques caractérisé en ce qu'il consiste à mélanger à des excipients convenables une dose efficace d'un dérivé du diméthyl-2,2,chromène préparé selon l'une des revendications 1 ou 2.

4. Procédé selon la revendication 3, pour la préparation de dose unitaire contenant de 0,5 à 200 mg d'un dérivé du diméthyl-2,2 chromène préparé selon l'une des revendications 1 ou 2.

**Revendications pour l'Etat contractant suivant : GR**

1. Dérivés du diméthyl-2,2 chromène de formule :

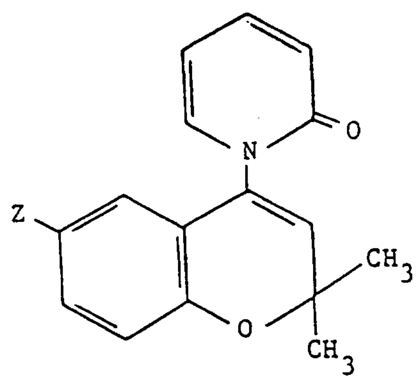

(I)

dans laquelle Z représente un groupement cyano, phosphono ou dialcoxyphosphoryle, le groupe alcoxy contenant 1 à 3 atomes de carbone; et les sels pharmaceutiquement acceptables du groupe phosphono.

2. Le cyano-6 (dihydro-1,2 oxo-2 pyridyl-1)-4 diméthyl-2,2 chromène.

3. Procédé de préparation d'un dérivé du chromène selon la revendication 1, caractérisé en ce que l'on déshydrate un chromannol-3 de formule :

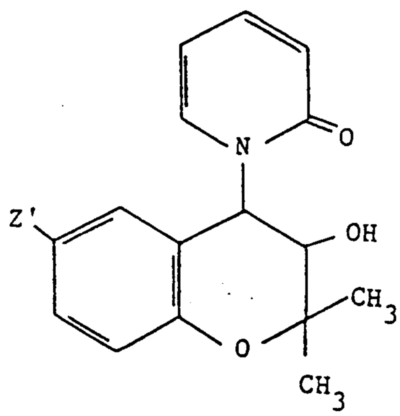

(II)

dans laquelle

- Z' représente un groupe cyano ou dialcoxyphosphoryle, par un hydrure alcalin et en ce qu'on transforme éventuellement le groupe dialcoxyphosphoryle en le groupe phosphono puis en ce qu'on transforme éventuellement l'acide phosponique ainsi obtenu en un de ses sels pharmaceutiquement acceptable.

**4.** Procédé pour la préparation de compositions pharmaceutiques caractérisé en ce qu'il consiste à mélanger à des excipients convenables une dose efficace d'un dérivé du diméthyl-2,2,chromène selon l'une des revendications 1 ou 2.

**5.** Procédé selon la revendication 4, pour la préparation de dose unitaire contenant de 0,5 à 200 mg d'un dérivé du diméthyl-2,2,chromène selon l'une des revendications 1 ou 2.

## Claims

## Claims for the following Contracting States : AT, BE, CH, DE, FR, GB, IT, LI, NL, SE

**1.** 2,2-Dimethylchromene derivatives of the formula :

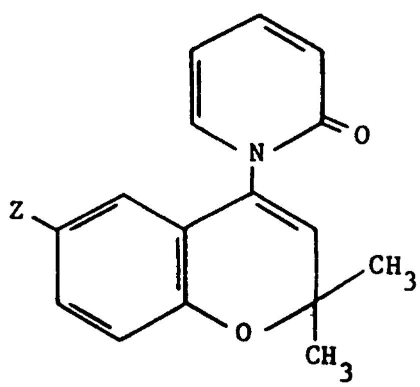

(I)

in which Z represents a phosphono or dialkoxyphosphoryl group, the alkoxy group containing 1 to 3 carbon atoms;
and the pharmaceutically acceptable salts of the phosphono group.

**2.** 6-Diethylphosphono-4-(1,2-dihydro-2-oxopyrid-1-yl)-2,2-dimethylchromene.

**3.** A process for the preparation of a chromene derivative according to claim 1, characterized in that it comprises dehydrating a chroman-3-ol of the formula :

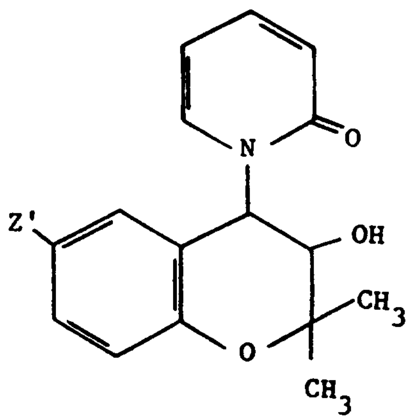

(II)

in which

- Z' represents a dialkoxyphosphoryl group, with an alkali metal hydride, and, if desired, converting the dialkoxyphosphoryl group into the phosphono group, and then, if desired, converting the resulting phosphonic acid into one of its pharmaceutically acceptable salts.

**4.** A pharmaceutical composition, characterized in that it contains, as the active principle, a 2,2-dimethylchromene derivative according to one of claims 1 or 2.

**5.** A pharmaceutical composition according to claim 4, containing from 0.5 to 200 mg of active principle per dosage unit, mixed with a pharmaceutical excipient.

**Claims for the following Contracting State : LU**

**1.** 2,2-Dimethylchromene derivatives of the formula :

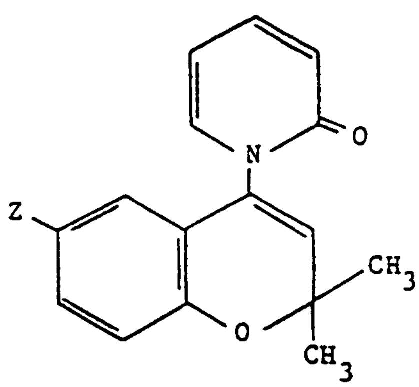

(I)

in which Z represents a cyano, phosphono or dialkoxyphosphoryl group, the alkoxy group containing 1 to 3 carbon atoms;
and the pharmaceutically acceptable salts of the phosphono group.

**2.** 6-Cyano-4-(1,2-dihydro-2-oxopyrid-1-yl)-2,2-dimethylchromene.

**3.** A process for the preparation of a chromene derivative according to claim 1, characterized in that it comprises dehydrating a chroman-3-ol of the formula :

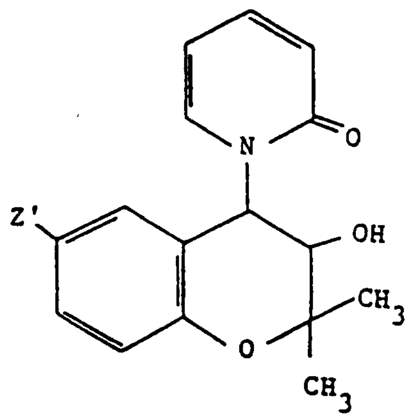

(II)

in which

- Z' represents a cyano or dialkoxyphosphoryl group, with an alkali metal hydride, and, if desired, converting the dialkoxyphosphoryl group into the phosphono group, and then, if desired, converting the resulting phosphonic acid into one of its pharmaceutically acceptable salts.

**4.** A pharmaceutical composition, characterized in that it contains, as the active principle, a 2,2-dimethylchromene derivative according to one of claims 1 or 2.

**5.** A pharmaceutical composition according to claim 4, containing from 0.5 to 200 mg of active principle per dosage unit, mixed with a pharmaceutical excipient.

**Claims for the following Contracting State : ES**

**1.** A process for the preparation of 2,2-dimethylchromene derivatives of the formula :

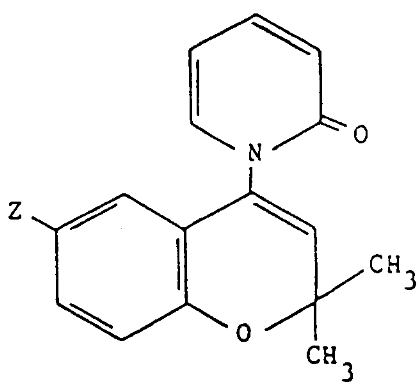

(I)

in which Z represents a phosphono or dialkoxyphosphoryl group, the alkoxy group containing 1 to 3 carbon atoms;
and the pharmaceutically acceptable salts of the phosphono group, characterized in that it comprises dehydrating a chroman-3-ol of the formula :

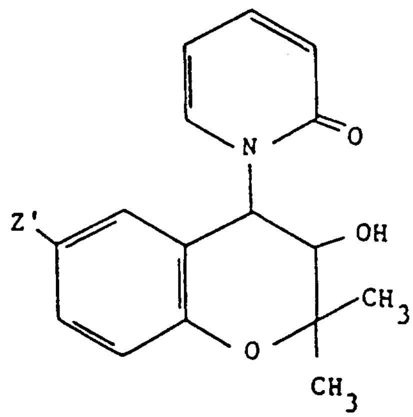

(II)

in which

- Z' represents a dialkoxyphosphoryl group, with an alkali metal hydride, and, if desired, converting the dialkoxyphosphoryl group into the phosphono group, and then, if desired, converting the resulting phosphonic acid into one of its pharmaceutically acceptable salts.

**2.** A process according to claim 1, for the preparation of 6-diethylphosphono-4-(1,2-dihydro-2-oxopyrid-1-yl)-2,2-dimethylchromene characterized in that it consists in dehydrating the chroman-3-ol of the formula :

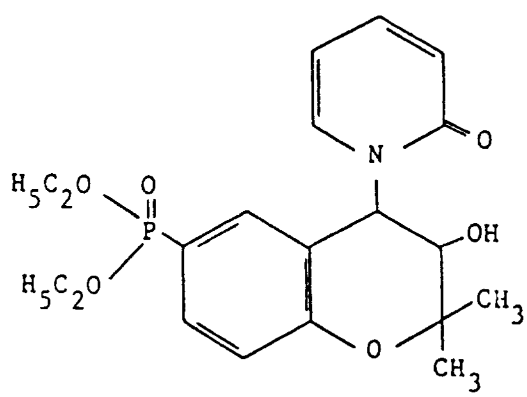

with sodium hydride.

**3.** A process for the preparation of pharmaceutical compositions, characterized in that it consists in mixing to suitable excipients an effective dose of a 2,2-dimethylchromene derivative according to one of claims 1 or 2.

**4.** A process according to claim 3, for the preparation of unit dosage containing from 0.5 to 200 mg of a 2,2-dimethylchromene derivative prepared according to one of claims 1 or 2.

**Claims for the following Contracting State : GR**

**1.** 2,2-Dimethylchromene derivatives of the formula :

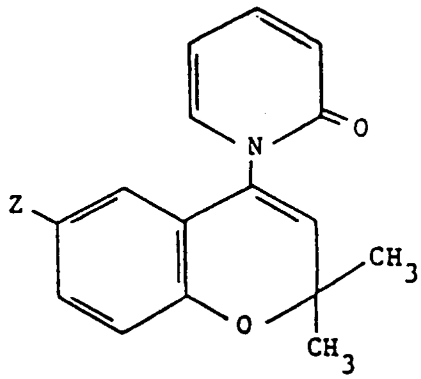

(I)

in which Z represents a cyano, phosphono or dialkoxyphosphoryl group, the alkoxy group containing 1 to 3 carbon atoms;
and the pharmaceutically acceptable salts of the phosphono group.

**2.** 6-Cyano-4-(1,2-dihydro-2-oxopyrid-1-yl)-2,2-dimethylchromene.

**3.** A process for the preparation of a chromene derivative according to claim 1, characterized in that it comprises dehydrating a chroman-3-ol of the formula :

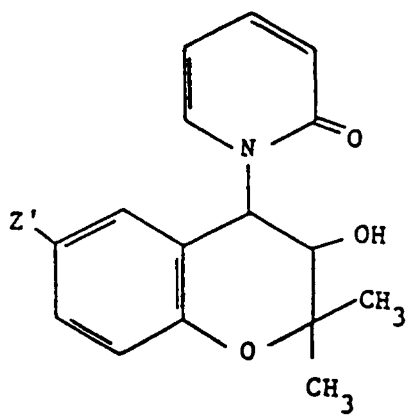

(II)

in which

- Z' represents a cyano or dialkoxyphosphoryl group, with an alkali metal hydride, and, if desired, converting the dialkoxyphosphoryl group into the phosphono group, and then, if desired, converting the resulting phosphonic acid into one of its pharmaceutically acceptable salts.

**4.** A process for the preparation of pharmaceutical compositions, characterized in that it consists in mixing to suitable excipients an effective dose of a 2,2-dimethylchromene derivative according to one of claims 1 or 2

**5.** A process according to claim 4, for the preparation of unit dosage containing from 0.5 to 200 mg of a 2,2-dimethylchromene derivative according to one of claims 1 or 2.

**Patentansprüche**
**Patentansprüche für folgende Vertragsstaaten : AT, BE, CH, DE, FR, GB, IT, LI, NL, SE**

1. 2,2-Dimethylchromenderivate der Formel:

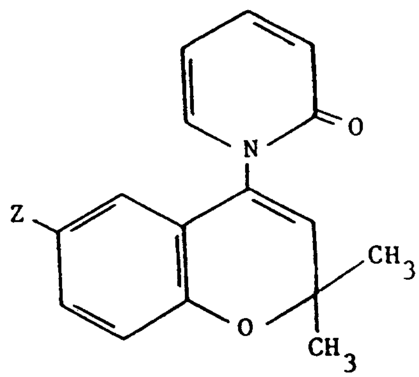

(I)

worin Z eine Phosphono- oder Dialkoxyphosphorylgruppe darstellt, wobei die Alkoxygruppe 1 bis 3 Kohlenstoffatome enthält; und die pharmazeutisch akzeptablen Salze der Phosphonogruppe.

2. 6-Diethylphosphono-4-(1,2-dihydro-2-oxo-1-pyridyl)-2,2-dimethylchromen.

3. Verfahren zur Herstellung eines Chromenderivats nach Anspruch 1, dadurch gekennzeichnet, daß man ein 3-Chromanol der Formel:

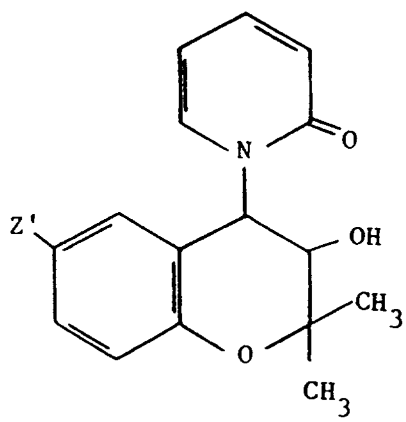

(II)

worin Z' eine Dialkoxyphosphorylgruppe darstellt, mit einem Alkalihydrid dehydratisiert, und daß man gegebenenfalls die Dialkoxyphosphorylgruppe in die Phosphonogruppe überführt, und daß man dann gegebenenfalls die so erhaltene Phosphonsäure in eines ihrer pharmazeutisch akzeptablen Salze überführt.

4. Pharmazeutische Zusammensetzung, dadurch gekennzeichnet, daß sie als Wirkstoff ein 2,2-Dimethylchromenderivat nach einem der Ansprüche 1 oder 2 enthält.

5. Pharmazeutische Zusammensetzung nach Anspruch 4, die 0,5 bis 200 mg Wirkstoff pro Dosiseinheit in Mischung mit einem pharmazeutischen Exzipienten enthält.

**Patentansprüche für folgenden Vertragsstaat : LU**

1. 2,2-Dimethylchromenderivate der Formel:

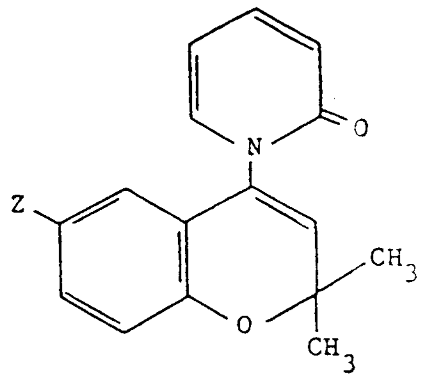

(I)

worin Z eine Cyano-, Phosphono- oder Dialkoxyphosphorylgruppe darstellt, wobei die Alkoxygruppe 1 bis 3 Kohlenstoffatome enthält; und die pharmazeutisch akzeptablen Salze der Phosphonogruppe.

2. 6-Cyano-4-(1,2-dihydro-2-oxo-1-pyridyl)-2,2-dimethylchromen.

3. Verfahren zur Herstellung eines Chromenderivats nach Anspruch 1, dadurch gekennzeichnet, daß man ein 3-Chromanol der Formel:

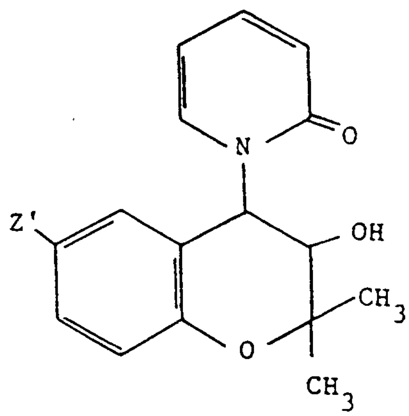

(II)

worin Z' eine Cyano- oder Dialkoxyphosphorylgruppe darstellt, mit einem Alkalihydrid dehydratisiert, und daß man gegebenenfalls die Dialkoxyphosphorylgruppe in die Phosphonogruppe überführt, und daß man dann gegebenenfalls die so erhaltene Phosphonsäure in eines ihrer pharmazeutisch akzeptablen Salze überführt.

4. Pharmazeutische Zusammensetzung, dadurch gekennzeichnet, daß sie als Wirkstoff ein 2,2-Dimethylchromenderivat nach einem der Ansprüche 1 oder 2 enthält.

5. Pharmazeutische Zusammensetzung nach Anspruch 4, die 0,5 bis 200 mg Wirkstoff pro Dosiseinheit in Mischung mit einem pharmazeutischen Exzipienten enthält.

**Patentansprüche für folgenden Vertragsstaat : ES**

1. Verfahren zur Herstellung von 2,2-Dimethylchromenderivaten der Formel:

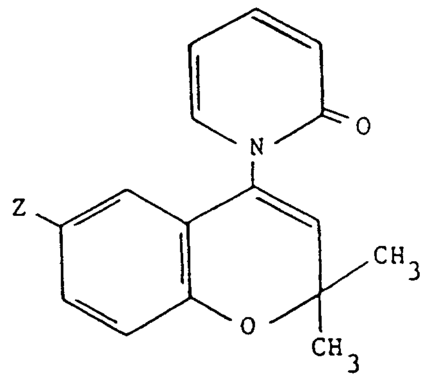

(I)

worin Z eine Phosphono- oder Dialkoxyphosphorylgruppe darstellt, wobei die Alkoxygruppe 1 bis 3 Kohlenstoffatome enthält; und der pharmazeutisch akzeptablen Salze der Phosphonogruppe, dadurch gekennzeichnet, daß man ein 3-Chromanol der Formel:

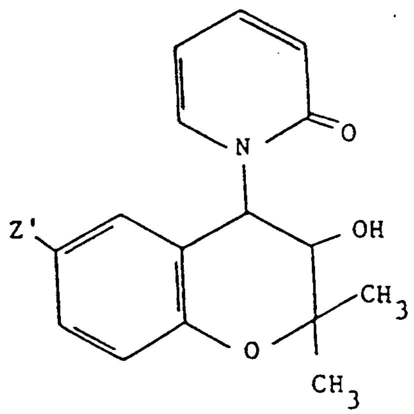

(II)

worin Z' eine Dialkoxyphosphorylgruppe darstellt, mit einem Alkalihydrid dehydratisiert, und daß man gegebenenfalls die Dialkoxyphosphorylgruppe in die Phosphonogruppe überführt, und daß man dann gegebenenfalls die so erhaltene Phosphonsäure in eines ihrer pharmazeutisch akzeptablen Salze überführt.

2. Verfahren nach Anspruch 1 zur Herstellung von 6-Diethylphosphono-4-(1,2-dihydro-2-oxo-1-pyridyl)-2,2-dimethylchromen, dadurch gekennzeichnet, daß es darin besteht, daß das 3-Chromanol der Formel:

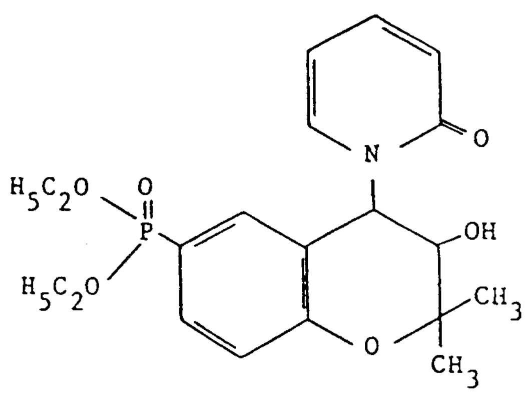

mit Natriumhydrid dehydratisiert wird.

3. Verfahren zur Herstellung von pharmazeutischen Zusammensetzungen, dadurch gekennzeichnet, daß es darin besteht, daß eine wirksame Dosis eines 2,2-Dimethylchromenderivats, hergestellt nach einem der Ansprüche 1 oder 2, mit geeigneten Exzipienten vermischt wird.

4. Verfahren nach Anspruch 3 zur Herstellung einer Einheitsdosis, die 0,5 bis 200 mg eines 2,2-Dimethylchromenderivats, hergestellt nach einem der Ansprüche 1 oder 2, enthält.

**Patentansprüche für folgenden Vertragsstaat : GR**

1. 2,2-Dimethylchromenderivate der Formel:

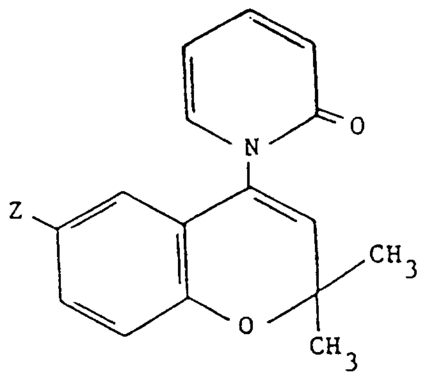

(I)

worin Z eine Cyano-, Phosphono- oder Dialkoxyphosphorylgruppe darstellt, wobei die Alkoxygruppe 1 bis 3 Kohlenstoffatome enthält; und die pharmazeutisch akzeptablen Salze der Phosphonogruppe.

2. 6-Cyano-4-(1,2-dihydro-2-oxo-1-pyridyl)-2,2-dimethylchromen.

3. Verfahren zur Herstellung eines Chromenderivats nach Anspruch 1, dadurch gekennzeichnet, daß man ein 3-Chromanol der Formel:

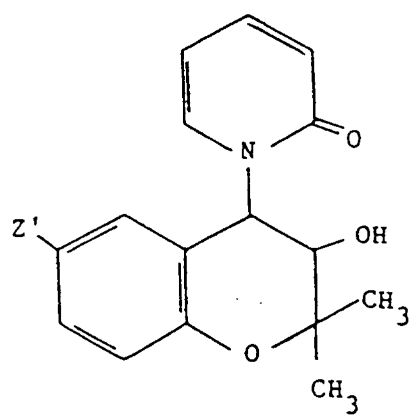

(II)

worin Z' eine Cyano- oder Dialkoxyphosphorylgruppe darstellt, mit einem Alkalihydrid dehydratisiert, und daß man gegebenenfalls die Dialkoxyphosphorylgruppe in die Phosphonogruppe überführt, und daß man dann gegebenenfalls die so erhaltene Phosphonsäure in eines ihrer pharmazeutisch akzeptablen Salze überführt.

4. Verfahren zur Herstellung von pharmazeutischen Zusammensetzungen, dadurch gekennzeichnet, daß es darin besteht, daß eine wirksame Dosis eines 2,2-Dimethylchromenderivats nach einem der Ansprüche 1 oder 2 mit geeigneten Exzipienten vermischt wird.

5. Verfahren nach Anspruch 4 zur Herstellung einer Einheitsdosis, die 0,5 bis 200 mg eines 2,2-Dimethylchromenderivats nach einem der Ansprüche 1 oder 2 enthält.